# EUROPÄISCHE PATENTSCHRIFT

(11) **EP 3 315 985 B1**
(45) Veröffentlichungstag und Bekanntmachung des Hinweises auf die Patenterteilung: **23.12.2020**
(21) Anmeldenummer: 16195656.0
(22) Anmeldetag: 26.10.2016
(51) Int. Cl.: G01R 33/28, G10K 11/178, H04R 1/10

(54) **MR-AUDIOEINHEIT**
MR AUDIO UNIT
UNITÉ AUDIO RM

(43) Veröffentlichungstag der Anmeldung: 02.05.2018
(73) Patentinhaber: Siemens Healthcare GmbH, 91052 Erlangen (DE)
(72) Erfinder: Popescu, Stefan, 91056 Erlangen (DE)

(56) Entgegenhaltungen:
- EP-B1- 2 095 144
- JP-A- 2014 200 609
- US-A- 5 552 708
- US-A1- 2003 103 638
- US-A1- 2005 197 565

## Beschreibung

Die Erfindung betrifft eine Audioeinheit zur Unterdrückung einer Wahrnehmung eines durch eine Magnetresonanzanlage verursachten Geräuschs, ein Audiosystem und ein Magnetresonanzsystem sowie ein Verfahren zum Betrieb einer Audioeinheit.

Die Magnetresonanztomographie (MRT; engl. Magnetic Resonance Imaging, MRI) ist eine bekannte Technik zum Erzeugen von Abbildungen eines Körperinneren eines Patienten, die auf dem physikalischen Phänomen der Magnetresonanz (MR) beruht. Während einer MR-Messung werden Gradientenspulen einer Gradientenspuleneinheit einer Magnetresonanzanlage mit hohen und kurzen Strompulsen betrieben, z.B. mit einer Amplitude von 600 Ampere mit Anstiegs- und Abfallzeiten von weniger als einer Mikrosekunde. Diese Strompulse erzeugen Magnetfeldgradienten, welche zur Enkodierung von Signalen verwendet werden, welche essentiell zur Erzeugung von Abbildungen ist.

Zudem erzeugt üblicherweise ein Hauptmagnet der Magnetresonanzanlage ein zeitlich konstantes Hauptmagnetfeld, dem die Magnetfeldgradienten überlagert werden. In diesem starken Hauptmagnetfeld erfahren die Leiter der Gradientenspulen, durch welche die Gradientenströme fließen, starke oszillierende Lorenz-Kräfte. Dadurch vibriert die Gradientenspuleneinheit und erzeugt ein lautes akustisches Geräusch (>100 dBA) innerhalb des Patientenaufnahmebereichs und des Untersuchungsraumes.

Dieses laute akustische Geräusch verursacht beim Patienten üblicherweise Unbehagen und Stress während der MR-Messung, insbesondere bei lang andauernden MR-Pulssequenzen. Dadurch kommt es oft zu einer Frustration des Patienten und zu unbeabsichtigten Bewegungen des Patienten, welche zu einer Reduktion der Bildqualität führen können.

Die Methoden zur Reduktion des akustischen Geräusches, welches das Hörorgan des Patienten während einer MR-Messung erreicht, können in passive und aktive Methoden unterteilt werden. Eine passive Methode reduziert das vom Patienten wahrgenommene Geräusch mit einer zusätzlichen akustischen Barriere. Diese Methoden schwächen den externen Schalldruck mit Hilfe von Ohrenschützern oder Gehörschutzstöpsel.

Alternative passive Methoden reduzieren Geräusche durch dämpfende Materialien oder luftleere Schallempfänger, um die Vibration der Magnetresonanzanlage an sich zu vermindern, insbesondere innerhalb des Patientenaufnahmebereichs, in dem der Patient gelagert wird.

Jedoch sind die Möglichkeiten der Geräuschreduktion mittels passiver Methoden eher gering. Sie sind zwar üblicherweise ausreichend, um den Schalldruck soweit abzusenken, dass das Hörorgan des Patienten nicht beschädigt wird, aber unzureichend, um die damit verbundenen Unannehmlichkeiten für den Patienten während der MR-Messung zu vermeiden.

Aktive Methoden ergänzen üblicherweise passive Methoden, indem sie das akustische Geräusch durch destruktive Interferenz weiter abschwächen. In der Regel verwenden diese Methoden einen externen Lautsprecher, der weit entfernt vom Patientenaufnahmebereich der Magnetresonanzanlage positioniert ist. Mit Hilfe eines Kunststoffschlauchs werden destruktiv interferierende Schallwellen vom Laufsprecher zum Patientenaufnahmebereich, insbesondere zum Ohr des Patienten, übertragen.

Jedoch leidet diese Methode unter Schallfeldstörungen und Signalverzögerungen aufgrund des langen akustischen Wegs, so dass insbesondere hochfrequente Geräuschanteile, insbesondere Anteile über 1 kHz, nicht effektiv ausgelöscht werden können. Des Weiteren sind üblicherweise die Mikrofone, mit denen das Geräusch innerhalb der Magnetresonanzanlage aufgenommen wird, vom Ohr des Patienten so weit beabstandet, dass nicht das tatsächlich im Ohr des Patienten auftretende Geräusch aufgenommen wird. Auch dies schränkt die Leistungsfähigkeit der Geräuschunterdrückung weiter ein.

Die Druckschrift US 2005/197565 A1 offenbart eine Vorrichtung, um eine bessere Kommunikation eines Patienten in einer Magnetresonanzanlage mit einem Bedienpersonal an einer Bedienkonsole zu ermöglichen. Dazu bedient man sich einer Knochensignalleitung.

Die Druckschrift JP 2014 200609 A offenbart eine Vorrichtung, um eine bessere Kommunikation eines Patienten in einer Magnetresonanzanlage mit einem Arzt trotz eines starker durch die Magnetresonanzanlage verursachter Geräusche ermöglichen. Um die Geräusche zu reduzieren, umfasst die Vorrichtung eine oder mehr geräuschisolierende Materialien.

Die Druckschrift EP 2 095 144 B1 offenbart eine Vorrichtung zur Geräuschunterdrückung. Die durch die Magnetresonanzanlage verursachten Geräusche werden dabei mittels eines ersten Mikrophons aufgenommen, das außerhalb des Gehörgangs angeordnet ist. Mit einem zweiten Mikrophon, das in der Nähe des Gehörgangs angeordnet ist, wird die Stimme des Patienten aufgenommen. Ferner umfasst die Vorrichtung einen Lautsprecher, der derart angetrieben wird, dass die Stimme des Patienten und die Geräusche unterdrückt werden.

Als Aufgabe der vorliegenden Erfindung kann angesehen werden, eine Vorrichtung und ein Verfahren anzugeben, das eine verbesserte Unterdrückung einer Wahrnehmung eines durch eine Magnetresonanzanlage verursachten Geräuschs ermöglicht.

Die Aufgabe wird durch die Merkmale der unabhängigen Ansprüche gelöst. Vorteilhafte Ausgestaltungen sind in den Unteransprüchen beschrieben.

Demnach wird eine Audioeinheit zur Unterdrückung einer Wahrnehmung eines durch eine Magnetresonanzanlage verursachten Geräuschs vorgeschlagen. Dabei umfasst die Audioeinheit eine Vibrationssensoreinheit, eine Steuereinheit, insbesondere einen Mikrocontroller, und zumindest einen Lautsprecher. Die die Vibrationssensoreinheit ist ausgebildet, mittels Knochenleitung übertragene Signale zu detektieren und an die Steuereinheit zu übertragen, wobei die Steuereinheit ausgebildet ist, anhand der übertragenen Signale ein destruktiv interferierendes Signal zu ermitteln und an den Lautsprecher auszugeben.

Ferner wird ein Verfahren zum Betrieb einer Audioeinheit vorgeschlagen, wobei in einer Betriebsphase einer Gradientenspuleneinheit einer Magnetresonanzanlage durch eine Vibrationssensoreinheit der Audioeinheit ein mittels Knochenleitung übertragenes Knochenleitungssignal detektiert wird, dadurch gekennzeichnet, dass anhand des Knochenleitungssignals durch zumindest einen Lautsprecher (S) der Audioeinheit (100) ein destruktiv interferierendes Lautsprechersignal erzeugt wird. Die Vibrationssensoreinheit ist ausgebildet, Signale zu detektieren, die über einen Festkörper in die Vibrationssensoreinheit einkoppelbar sind, d.h. die Vibrationssensoreinheit ist vorzugsweise nicht dazu ausgelegt, Luftschall, also Druckschwingungen der Luft, zu erfassen, sondern Körperschall. Das durch die Vibrationssensoreinheit detektierbare Signal ist also eine mechanische Schwingung, insbesondere eine mechanische Vibration, die nicht mittels Luftleitung, also mittels einer Übertragung über ein gasförmiges Medium, auf die Vibrationssensoreinheit übertragen wird, sondern mittels eines festen Mediums.

Die Vibrationssensoreinheit ist vorzugsweise ausgebildet, mechanische Vibrationen zu detektieren, die von einer Magnetresonanzanlage, insbesondere von einer Gradientenspuleneinheit einer Magnetresonanzanlage erzeugt werden. In der Regel werden diese mechanischen Vibrationen im Wesentlichen über nicht-gasförmige, also insbesondere feste, Medien zwischen der Quelle der Vibrationen, insbesondere Gradientenspulen, zu der Vibrationssensoreinheit übertragen. Beispielsweise werden die Vibrationen von Gradientenspulen während einer MR-Messung erzeugt, von dort aus über ein Gehäuse der Magnetresonanzanlage auf einen Patiententisch übertragen. Über den Patiententisch kann die Vibration über eine Knochenleitung des Patienten, beispielsweise über den Schädel, das Schläfenbein (lat. Os temporale) und/oder die Wände des Gehörgangs, zu dem Hörorgan des Patienten, insbesondere zu den Gehörknöchelchen (lat. Ossicula auditüs) und der Hörschnecke (lat. Cochlea), übertragen werden.

Die auf diese Weise erfolgte Anregung der der Hörschnecke ist im Wesentlichen unabhängig von den durch Luftleitung übertragenen Signalen durch den Gehörgang (lat. Meatus acusticus), die mit Hilfe des Trommelfells (lat. Membrana tympani) auf die Gehörknöchelchen eingeleitet werden.

Da ein großer Anteil der Störgeräusche, die durch eine Magnetresonanzanlage verursacht werden, über Knochenleitung auf den Patienten einwirken, können anhand der Signale, die durch die Vibrationssensoreinheit detektiert werden, effektivere Gegenmaßnahmen zur Geräuschunterdrückung durchgeführt werden.

Die Steuereinheit ermittelt anhand der mittels Knochenleitung übertragenen Signale ein destruktiv interferierendes Signal, das über den Lautsprecher ausgegeben wird. Dabei ist der Lautsprecher vorzugsweise MR-kompatibel, insbesondere nichtmagnetisch. Somit kann insbesondere die Wahrnehmung des Geräuschs, das durch Knochenleitung übertragen wird, durch den Patienten reduziert oder sogar ausgelöscht werden.

Eine Ausführungsform der Audioeinheit sieht vor, dass die Vibrationssensoreinheit zumindest einen Inertialsensor und/oder zumindest einen Beschleunigungssensor und/oder zumindest einen Drehratensensor umfasst. Diese Sensortypen eignen sich besonders, um mittels Knochenleitung übertragene Signale zu detektieren.

Beschleunigungssensoren werden oftmals auch Beschleunigungsmesser, Akzelerometer, B-Messer und/oder G-Sensor genannt. Beschleunigungssensoren messen meistens eine Beschleunigung, indem eine auf eine Testmasse wirkende Trägheitskraft bestimmt wird. Somit kann z. B. bestimmt werden, ob eine Geschwindigkeitszunahme oder -abnahme stattfindet. Aufgrund dieses üblichen Wirkprinzips können Beschleunigungssensoren, wie auch die Drehratensensoren, in der Regel der Gruppe der Inertialsensoren zugeordnet werden.

Bevorzugt umfasst die Vibrationssensoreinheit zumindest einen Mehrachsen-Beschleunigungssensor, z. B. einen Dreiachsen-Beschleunigungssensor. Damit können beispielsweise drei Signale erfasst werden, die zueinander orthogonale Komponenten eines Gesamtsignals sind. So kann beispielsweise eine x-Komponente in einer x-Richtung, eine y-Komponente in einer y-Richtung und eine z-Komponente in einer z-Richtung erfasst werden, wobei x-, y- und z-Richtung jeweils senkrecht zueinander orientiert sind.

Somit kann die mittels Knochenleitung übertragene Signal richtungsabhängig detektiert werden, d.h. es kann beispielsweise nicht nur eine Amplitude einer Vibration bestimmt werden, sondern auch eine Ausbreitungsrichtung der Vibration, d. h. die Richtung, aus der die Vibration zu der Vibrationssensoreinheit kommt. Diese zusätzliche Information kann die Auswertemöglichkeiten des detektierten Signals durch die Steuereinheit erweitern.

Vorzugsweise ist die Steuereinheit ausgebildet, aus dem mit dem Mehrachsen-Beschleunigungssensor detektierbaren Signal zumindest zwei Signalanteile verschiedener Ausbreitungsrichtungen zu separieren. Vorzugsweise erfasst der Mehrachsen-Beschleunigungssensor für jeder seiner mehreren Achsen ein eine Signalkomponente, wobei die die Separation anhand von Amplituden und/oder Verzögerungszeiten der Signalkomponenten erfolgt. Diese Separation kann beispielsweise mit Hilfe einer Kreuzkorrelationsanalyse erfolgen.

Da sich beispielsweise die Ausbreitungsrichtung einer Vibration im Schädel und/oder im Schläfenbein abhängig von der Quelle der Vibration unterscheidet, können Signalanteile unterschiedlicher Quellen aus dem (Gesamt-)Signal separiert werden.

Beispielsweise kann eine erste Vibrationsquelle die Stimmlippen (lat. Plica vocalis) des Patienten sein und eine weitere Vibrationsquelle eine Oberfläche eines Patiententisches, auf der der Patient liegt. Vibrationen, die durch die Stimmlippen des Patienten verursacht werden, z. B. indem der Patient spricht, können sich also unterschiedlich bezüglich einer x-, y- und z-Richtung ausbreiten verglichen mit Vibrationen der Oberfläche des Patiententisches. Damit weisen die x-, y- und z-Komponenten des durch den Beschleunigungssensor erfassten Signals spezifische Amplituden und/oder Verzögerungszeiten auf abhängig vom Ort der Vibrationsquelle. Durch eine Separation des durch die Vibrationssensoreinheit detektierten Signals können in diesem Beispiel gezielt die störenden Vibrationen unterdrückt werden, die vom Patiententisch ausgehen, während die Vibrationen ausgehend von den Stimmlippen nicht unterdrückt werden, so dass sich der Patient selbst sprechen hört.

Vorzugsweise ist die Vibrationssensoreinheit ausgebildet, mechanische Schwingungen in einem Frequenzbereich zwischen 15 Hz und 20 kHz, insbesondere zwischen 15 Hz und 1 kHz, zu detektieren. Gerade in diesem Frequenzbereich werden üblicherweise durch eine Magnetresonanzanlage besonders starke und/oder störende Vibrationen erzeugt, die über Knochenleitung übertragen werden.

Eine Ausführungsform der Audioeinheit sieht vor, dass die Audioeinheit einen ersten Abschnitt umfasst, der eine Form aufweist, die geeignet ist, den ersten Abschnitt in einen, insbesondere äußeren, Gehörgang eines, insbesondere menschlichen, Patienten einzuführen, wobei die Vibrationssensoreinheit im ersten Abschnitt der Audioeinheit angeordnet ist. Vorteilhafterweise ist die Form des ersten Abschnitts darüber hinaus auch geeignet, einen festen Sitz des ersten Abschnitts im Gehörgang sicherzustellen. Dazu kann der erste Abschnitt ein oder mehrere elastische Elemente aufweisen, die sich der Form des Gehörgangs anpassen können.

Bevorzugt weist die Vibrationssensoreinheit eine erste äußere Grenzfläche auf, die die Audioeinheit nach außen begrenzt und/oder die Audioeinheit weist ein schallleitendes Material mit einer zweiten äußeren Grenzfläche aufweist, das zwischen der Vibrationssensoreinheit und der zweiten äußeren Grenzfläche angeordnet ist, so dass die erste äußere Grenzfläche und/oder zweite äußere Grenzfläche in einem eingeführten Zustand eine Wand des Gehörgangs berührt.

Durch eine Anordnung der Vibrationssensoreinheit in, insbesondere rigidem, mechanischen Kontakt mit dem Gewebe des Gehörgangs können die mittels Knochenleitung übertragenen Signale besonders gut auf die Vibrationssensoreinheit übertragen und/oder von diesem detektiert werden.

Die Audioeinheit kann vorzugsweise in Form eines Kopfhörers, insbesondere eines Ohrhöhrers ausgebildet sein. Insbesondere kann die Audioeinheit in Form eines Gehörganghörer und/oder eines Im-Ohr-Kopfhörers (engl. In-ear headphones) und/oder eines Earbud-Ohrhörer und/oder eines Halb-im-Ohr-Kopfhörers (engl. Half-In-ear-headphones) ausgebildet sein.

Diese Bauformen ermöglichen eines kompakte Bauweise und einen hohen Patientenkomfort. Vorteilhafterweise schränken diese Bauformen die Bewegungsfreiheit, insbesondere die Rotationsmöglichkeiten, des Kopfs im Vergleich zu größeren Bauformen, beispielsweise derjenigen eines Muschelkopfhörers, nicht oder nur wenig ein.

Vorzugsweise ist die Audioeinheit kleiner als 5 cm, insbesondere kleiner als 3 cm. Insbesondere sind die elektrischen Teile der Audioeinheit in einem Gehäuse angeordnet, das eine Ausdehnung von weniger als 5 Zentimeter, insbesondere weniger als 3 cm, aufweist. Dadurch ist die maximale Größe und/oder Länge etwaiger elektrischer Drähte und/oder Antennen und/oder Leitungen innerhalb der Audioeinheit deutlich kleiner als die Wellenlänge der Hochfrequenz-Strahlung, die bei einer MR-Messung üblicherweise verwendet wird. Somit wird das Risiko reduziert, große elektrische Felder zu induzieren und/oder die Audioeinheit während einer MR-Messung zu stark aufzuheizen.

Eine weitere Ausführungsform sieht vor, dass die Audioeinheit zumindest ein, insbesondere MR-kompatibles, Mikrofon umfasst, das ausgebildet ist, mittels Luftleitung übertragene Signale zu detektieren und an die Steuereinheit zu übertragen.

Unter einem Mikrofon ist also üblicherweise ein Schallwandler zu verstehen, der Luftschall als Schallwechseldruckschwingungen in entsprechende elektrische Spannungsänderungen als Mikrofonsignal umwandelt.

Vorzugsweise umfasst das zumindest eine Mikrofon ein Kondensatormikrofon, insbesondere ein Elektret-Kondensatormikrofon (engl. Electret Condenser Microphone, ECM). Ein Elektret ist ein elektrisch isolierendes Material, das quasi-permanent gespeicherte elektrische Ladungen oder quasi-permanent ausgerichtete elektrische Dipole enthält und somit ein quasipermanentes elektrisches Feld in seiner Umgebung oder in seinem Inneren erzeugt. Dadurch entfällt bei einem ECM vorteilhafterweise die Notwendigkeit einer polarisierenden Spannungsversorgung.

Vorzugsweise umfasst die Audioeinheit ein inneres Mikrofon, das im ersten Abschnitt der Audioeinheit angeordnet ist, der - wie bereits beschrieben - eine Form aufweist, die geeignet ist, den ersten Abschnitt in einen Gehörgang einzuführen. Das innere Mikrofon eignet sich besonders gut, um Schall, der in den Gehörgang, insbesondere den äußeren Gehörgang, gelangt, zu detektieren.

Bevorzugt umfasst die Audioeinheit neben einem ersten Abschnitt, einen zweiten Abschnitt, der bei Einführung des ersten Abschnitts in den Gehörgang außerhalb des Gehörgangs angeordnet ist. Da innerhalb des Gehörgangs nur beschränkte Platzverhältnisse gegeben sind, kann mit Hilfe des zweiten Abschnitts ein erweiterter Bauraum für etwaige Komponenten der Audioeinheit zur Verfügung gestellt werden.

Vorzugsweise umfasst die Audioeinheit ein äußeres Mikrofon, das im zweiten Abschnitt der Audioeinheit angeordnet ist. Das äußere Mikrofon ist durch seine Anordnung besonders geeignet, äußere Störgeräusche und/oder die Stimme des Patienten zu detektieren.

Wie schon beschrieben, sind das innere und/oder das äußere Mikrofon vorzugsweise ausgebildet, mittels Luftleitung übertragene Signale zu detektieren und an die Steuereinheit zu übertragen.

Bevorzugt umfasst das äußere Mikrofon ein Mikrofonarray. Ein Mikrofonarray, das mitunter auch als akustische Kamera und/oder akustische Antenne bezeichnet wird, umfasst üblicherweise mehrere Mikrofonelemente, die insbesondere Beamforming ermöglichen. Dabei kann durch eine phasenrichtige Überlagerung von Einzelsignalen der Mikrofonelemente beispielsweise eine gewünschte Richtwirkung des Mikrofonarrays eingestellt werden, die idealerweise zudem auch elektronisch geschwenkt werden kann. Insbesondere kann damit je nach Situation das äußere Mikrofon so angepasst werden, dass es für die Situation optimierte Richtcharakteristik aufweist.

Vorzugsweise umfasst die Audioeinheit eine erste drahtlose Übertragungseinheit, die ausgebildet ist, elektromagnetische Signale zu detektieren und an die Steuereinheit übertragen und/oder von der Steuereinheit empfangene Signale als elektromagnetische Signale auszusenden.

Mit der ersten drahtlosen Übertragungseinheit können insbesondere Informationen zwischen der der Audioeinheit und einer etwaigen externen Einheit, beispielsweise einer Magnetresonanzanlage und/oder einer weiteren Audioeinheit, übertragen werden. Dabei umfasst die erste drahtlose Übertragungseinheit vorzugsweise zumindest eine Antenne, mit der die elektromagnetischen Signale gesendet und/oder empfangen werden können.

Die drahtlose Übertragungseinheit verwendet vorzugsweise ein bereits bestehendes Protokoll, das nicht mit MR-Frequenzen interferiert, um eine elektromagnetische Kompatibilität der Audioeinheit mit der MR-Umgebung zu erreichen. Beispielsweise arbeitet die drahtlose Übertragungseinheit in dem 2,4 GHz-Band, insbesondere dem 2,4-GHz-ISM-Band (Industrial, Scientific and Medical Band), das weltweit nicht lizensiert, aber dennoch nicht ungeregelt ist. Vorzugsweise wird ein standardisiertes Hochfrequenzprotokoll, wie Bluetooth, verwendet, das die Hochfrequenztechnologie Frequency Hopping Spread Spectrum (FHSS) einsetzt. Damit können Interferenzen mit den MR-Frequenzen vermieden werden.

Bei den genannten Frequenzen kann die Audioeinheit effizient gegen Hochfrequenzeffekte abgeschirmt werden, die durch den Betrieb der Magnetresonanzanlage hervorgerufen werden. Ferner können durch selektive Frequenzfilterung Wirbelströme, die durch das Schalten der Gradientenfelder erzeugt werden, signifikant reduziert werden.

Bevorzugt umfasst die Audioeinheit zumindest einen Energiespeicher. Dieser ist vorzugsweise ausgebildet, die Steuereinheit und/oder die erste drahtlose Übertragungseinheit und/oder möglicherweise weitere Komponenten der Audioeinheit mit Energie zu versorgen.

Der zumindest eine Energiespeicher kann beispielsweise einen wiederaufladbaren Akkumulator, insbesondere in weitgehend nichtmagnetischer Ausführung, und/oder eine Batterie, insbesondere eine Langzeit-Batterie, und/oder einen Kondensator, insbesondere als Doppelschichtkondensator mit hoher Kapazität, umfassen.

Der Energiespeicher sorgt idealerweise für einen durchgehenden Betrieb der Audioeinheit. Vorzugsweise ist der Energiespeicher drahtlos aufladbar, beispielsweise durch Energy Harvesting der Hochfrequenzleistung, die durch Hochfrequenzspulen der Magnetresonanzanlage abgestrahlt wird.

Ferner werden ein Audiosystem mit einer ersten Audioeinheit und eine zweite Audioeinheit vorgeschlagen. Dabei ist die ersten Audioeinheit beispielsweise für ein linkes Ohr eines Patienten ausgebildet und die zweite Audioeinheit für ein rechtes Ohr des Patienten. Die erste und die zweite Audioeinheit sind vorzugsweise zueinander symmetrisch geformt.

Vorzugsweise sind die erste Audioeinheit und die zweite Audioeinheit ausgebildet, miteinander zu kommunizieren. Bevorzugt umfassen beide Audioeinheiten jeweils eine drahtlose Übertragungseinheit, über welche elektromagnetische Signale zwischen den Audioeinheiten ausgetauscht werden können.

Insbesondere kann vorgesehen sein, dass die erste Audioeinheit eine erste drahtlose Übertragungseinheit umfasst, die ausgebildet ist, elektromagnetische Signale zu senden, wobei die zweite Audioeinheit eine zweite drahtlose Übertragungseinheit umfasst, die ausgebildet ist, elektromagnetische Signale zu senden, wobei die erste drahtlose Übertragungseinheit ausgebildet ist, von der zweiten drahtlosen Übertragungseinheit gesendete Signale zu empfangen, wobei die zweite drahtlose Übertragungseinheit ausgebildet ist, von der ersten drahtlosen Übertragungseinheit gesendete Signale zu empfangen.

So kann beispielsweise ein Signal, das einer oder mehrere der Sensoren, insbesondere die Vibrationssensoreinheit und/oder das innere Mikrofon und/oder das äußere Mikrofon, detektiert, von der ersten Audioeinheiten auf die zweite Audioeinheit übertragen werden und dort zur Ansteuerung des zumindest einen Lautsprecher der zweiten Audioeinheit verwendet werden.

Ferner wird ein Magnetresonanzsystem mit einer Magnetresonanzanlage, einem Audiosystem und/oder einer Audioeinheit vorgeschlagen. Bevorzugt sind die Magnetresonanzanlage, das Audiosystem und/oder die Audioeinheit ausgebildet, miteinander zu kommunizieren. Somit kann beispielsweise ein Bediener der Magnetresonanzanlage mit dem Patienten sprechen.

Dabei umfasst die Magnetresonanzanlage vorzugsweise eine weitere drahtlose Übertragungseinheit und eine Anlagensteuereinheit, wobei die weitere drahtlose Übertragungseinheit ausgebildet ist, elektromagnetische Signale zu detektieren und an die Anlagensteuereinheit übertragen und/oder von der Anlagensteuereinheit empfangene Signale als elektromagnetische Signale zu senden.

Im Weiteren werden verschiedene Ausführungsformen von Verfahren zum Betrieb einer Audioeinheit beschrieben, die mit der vorab beschriebenen Audioeinheit und/oder dem Audiosystem und/oder dem Magnetresonanzsystem ausgeführt werden können. Das Verfahren ist also nicht auf den Betrieb einer einzelnen Audioeinheit beschränkt, sondern kann auch auf ein Audiosystem mit mehreren Audioeinheiten, insbesondere einem Audiosystem mit einer Audioeinheit für das linke Ohr und eine weitere Audioeinheit das rechte Ohr eines Patienten, übertragen werden.

Die Vorteile dieser Vorrichtungen, die vorab im Detail erläutert wurden, können auf die im Weiteren beschriebenen Ausführungsformen der Verfahren übertragen werden und umgekehrt. Mit anderen Worten können die gegenständlichen Ansprüche auch mit den Merkmalen, die in Zusammenhang mit einem Verfahren beschrieben und/oder beansprucht sind, weitergebildet sein.

Es wird ein Verfahren zum Betrieb einer Audioeinheit vorgeschlagen, wobei die Audioeinheit in Abhängigkeit eines Betriebszustands einer Magnetresonanzanlage, insbesondere einer Gradientenspuleneinheit der Magnetresonanzanlage, betrieben, insbesondere in verschiedenen Betriebsarten, wird. Damit können die Möglichkeiten, die eine derartige Audioeinheit bietet, je nach Situation optimal ausgenutzt werden.

Beispielsweise können die verschiedenen Betriebsarten zumindest einen Sprachkommunikationsmodus und/oder einen Geräuschunterdrückungsmodus umfassen. Während im Geräuschunterdrückungsmodus möglichst effektiv das für den Patienten unangenehme Geräusch der Magnetresonanzanlage während des Messbetriebs unterdrückt wird, eignet sich der Sprachkommunikationsmodus vornehmlich zur Kommunikation zwischen einem Bediener der Magnetresonanzanlage und dem Patienten, der sich in der Magnetresonanzanlage befindet.

Bevorzugt wird in einer Ruhephase der Gradientenspuleneinheit der Magnetresonanzanlage ein akustisches Signal durch ein Mikrofon, insbesondere durch ein äußeres Mikrofon, der Audioeinheit detektiert, durch eine drahtlose Übertragungseinheit der Audioeinheit an eine drahtlose Übertragungseinheit der Magnetresonanzanlage übertragen und durch eine Ausgabeeinheit ausgegeben und/oder es wird ein Signal durch die drahtlose Übertragungseinheit der Magnetresonanzanlage an die drahtlose Übertragungseinheit der Audioeinheit übertragen und durch einen Lautsprecher der Audioeinheit ausgegeben.

Dabei kann die Ruhephase der Gradientenspuleneinheit ein Zeitraum sein, in dem die Gradientenspuleneinheit nicht betrieben wird. Insbesondere kann die Ruhephase der Gradientenspuleneinheit ein Zeitraum sein, in dem durch Gradientenspulen der Gradientenspuleneinheit kein Gradientenstrom, insbesondere kein gepulster Gradientenstrom, fließt und/oder durch die Gradientenspuleneinheit keine Magnetfeldgradienten erzeugt werden.

Mit einer Ruhephase sind hier üblicherweise nicht die kurzen Zeitintervalle innerhalb einer MR-Pulssequenz gemeint, in denen gerade kein Gradientenpuls appliziert wird, welche kurzen Zeitintervalle oft in der Größenordnung der Repetitionszeit TR liegen. Vorzugsweise ist ein solcher Zeitraum länger als 1, insbesondere länger als 10, Sekunden. Eine Ruhephase kann beispielsweise vor und/oder einer MR-Messung und/oder zwischen verschiedenen Abschnitten einer MR-Messung auftreten.

Ein derartiges Verfahren kann vorteilhafterweise in einem Sprachkommunikationsmodus durchgeführt werden. Der Patienten und der Bediener können somit auf komfortable Weise miteinander sprechen.

Bevorzugt wird zur Unterdrückung einer Wahrnehmung eines durch die Magnetresonanzanlage verursachten Geräuschs in einer Betriebsphase der Gradientenspuleneinheit der Magnetresonanzanlage durch zumindest einen Lautsprecher der Audioeinheit ein Lautsprechersignal erzeugt.

Der zumindest eine Lautsprecher wird dabei vorzugsweise durch eine Steuereinheit angesteuert, wie sie vorab bereits beschrieben wurde. Für eine geeignete Ansteuerung des zumindest einen Lautsprechers verwendet die Steuereinheit vorteilhafterweise Signale, die von etwaigen Sensoren, insbesondere der Vibrationssensoreinheit und/oder von Mikrofonen, detektiert und an die Steuereinheit übertragen werden.

Die Betriebsphase der Gradientenspuleneinheit kann ein Zeitraum sein, in dem die Gradientenspuleneinheit betrieben wird. Insbesondere kann die Ruhephase der Gradientenspuleneinheit einen oder mehrere Zeiträume umfassen, in dem durch die Gradientenspulen ein Gradientenstrom, insbesondere ein gepulster Gradientenstrom, fließt und/oder durch die Gradientenspuleneinheit Magnetfeldgradienten erzeugt werden.

Eine Betriebsphase kann aber auch Pausen umfassen, in denen gerade kein Gradientenstrom fließt und/oder durch die Gradientenspuleneinheit keine Magnetfeldgradienten erzeugt werden. Derartige Pause können durch die MR-Pulssequenz vorgegeben werden, die während der Betriebsphase durchgeführt wird. Eine MR-Pulssequenz legt üblicherweise eine Abfolge von Gradientenpulsen und weiteren Ereignissen, wie z. B. Hochfrequenzpulsen, fest. Zwischen zwei aufeinanderfolgenden Gradientenpulsen können daher Pausen auftreten, in denen kein Gradientenstrom und/oder durch die Gradientenspuleneinheit keine Magnetfeldgradienten erzeugt werden. Oftmals weist eine derartige Pause eine Zeitdauer auf, die in der Größenordnung der Repetitionszeit TR einer MR-Pulssequenz liegt. Somit sind derartige Pausen üblicherweise kleiner als 1 Sekunde, insbesondere kleiner als 0,1 Sekunden.

Vorzugsweise interferiert das Lautsprechersignal mit den Schallwellen, die auf das Gehörorgan des Patienten einwirken, derart, dass die Schallwellen, insbesondere die Amplitude der Schallwellen, reduziert wird. Wie bereits beschrieben kann diese Technik auch als destruktive Interferenz bezeichnet werden. Die resultierende empfundene Lärmverminderung wirkt sich in der Regel positiv auf das Wohlbefinden des Patienten aus.

Erfindungsgemäss wird durch eine Vibrationssensoreinheit der Audioeinheit ein mittels Knochenleitung übertragenes Knochenleitungssignal detektiert, wobei die Erzeugung des Lautsprechersignals anhand des Knochenleitungssignals erfolgt. Vorteilhafterweise erfolgt dabei die Erzeugung des Lautsprechersignals anhand einer Minimierung des Knochenleitungssignals.

Bei den auf das Gehörorgan des Patienten einwirkenden Schallwellen kann es sich also um per Knochenleitung übertragene Signale handeln, die auf diese Weise besonders effektiv unterdrückt werden können. Die Lautsprecher werden dabei vorzugsweise so durch die Steuereinheit der Audioeinheit angesteuert, dass die auf das Hörorgan des Patienten einwirkenden Vibrationen reduziert werden, die ansonsten als akustischer Lärm durch den Patienten wahrgenommen werden würden.

Bevorzugt werden dabei niederfrequente Anteile, insbesondere Anteile kleiner 1 kHz, des durch die Magnetresonanzanlage verursachten Geräuschs unterdrückt, da in diesem Frequenzbereich das Verfahren besonders effektiv ist.

Eine besonders vorteilhafte Ausführungsform des Verfahrens sieht vor, dass die Erzeugung des Lautsprechersignals anhand einer Zuordnungstabelle erfolgt, die empirisch ermittelte Vibrationsmuster umfasst, denen Kompensationsmuster zugeordnet sind. Die Zuordnungstabelle wird vorzugsweise a priori anhand von Labormessungen, z.B. an Versuchspersonen, kalibriert.

Vorzugsweise umfasst das Knochenleitungssignal mehrere richtungsabhängige Knochenleitungssignale, wobei das Knochenleitungssignal in Abhängigkeit der mehreren richtungsabhängigen Knochenleitungssignale in zumindest zwei Anteile, insbesondere in einen Sprachanteil und einen Geräuschanteil, separiert wird.

Die mehreren richtungsabhängigen Knochenleitungssignale können - wie bereits erläutert - mit Hilfe eines Mehrachsen-Beschleunigungssensors erfasst werden.

Eine Ausführungsform sieht vor, dass in einer Ruhephase der Gradientenspuleneinheit der Magnetresonanzanlage durch die Stimme eines Patienten Knochenleitungssignale erzeugt werden, die durch die Vibrationssensoreinheit, insbesondere als Lerndaten, detektiert werden und anhand derer in einer Betriebsphase des Gradientenspuleneinheit der Magnetresonanzanlage das Knochenleitungssignal in die zumindest zwei Anteile separiert werden.

Insbesondere werden während einer Ruhephase der Gradientenspuleneinheit, insbesondere in einem Sprachkommunikationsmodus, durch eine Stimme eines Patienten verursachte, insbesondere richtungsabhängige, Knochenleitungssignale erfasst, beispielsweise mit Hilfe des Mehrachsen-Beschleunigungssensors. Diese Knochenleitungssignale werden an die Steuereinheit übertragen, die auf diese Weise charakteristische Amplituden und/oder charakteristische Verzögerungszeiten, insbesondere bezüglich der mehreren Achsen des Mehrachsen-Beschleunigungssensors, lernen kann, die charakteristisch für die Stimme des Patienten sind.

In einer späteren Betriebsphase der Gradientenspuleneinheit, insbesondere in einem Geräuschunterdrückungsmodus, kann die Steuereinheit anhand der charakteristischen Amplituden und/oder charakteristischen Verzögerungszeiten die, insbesondere richtungsabhängigen, Knochenleitungssignale in zumindest zwei Anteile, insbesondere in einen Sprachanteil und einen Geräuschanteil, separieren. Der zumindest eine Lautsprecher kann so angesteuert werden, dass der Geräuschanteil unterdrückt wird, der Sprachanteil jedoch nicht.

In einer weiteren Ausführungsform können die charakteristischen Amplituden und/oder charakteristischen Verzögerungszeiten von einer ersten Audioeinheit auf eine zweite Audioeinheit ausgetauscht werden, beispielsweise mittels drahtloser Übertragungseinheiten, um die Separierung des Knochenleitungssignals weiter zu verbessern.

Vorzugsweise wird in einer Betriebsphase der der Gradientenspuleneinheit durch ein, insbesondere inneres, Mikrofon der Audioeinheit ein inneres akustisches Signal innerhalb eines Gehörgangs detektiert, wobei die Erzeugung des Lautsprechersignals anhand des inneren akustischen Signals erfolgt. Vorzugsweise erfolgt die Erzeugung des Lautsprechersignals anhand einer Minimierung des inneren akustischen Signals.

Das innere akustische Signal wird vorzugsweise an die Steuereinheit der Audioeinheit übertragen, welche das innere akustische Signal verarbeitet und den zumindest einen Lautsprechersignals zur Erzeugung des Lautsprechersignals ansteuert.

Damit können auch effektiv mittels Luftleitung übertragene Signale unterdrückt werden. Der zumindest eine Lautsprecher wird vorzugsweise so durch die Steuereinheit der Audioeinheit angesteuert, dass die durch den Gehörgang auf das Ohr des Patienten einwirkenden Luftschallwellen reduziert werden, die ansonsten als akustischen Lärm durch den Patienten wahrgenommen werden würden.

Ferner wird vorgeschlagen, dass in einer Betriebsphase der Gradientenspuleneinheit durch ein, insbesondere äußeres, Mikrofon der Audioeinheit ein äußeres akustisches Signal außerhalb eines Gehörgangs detektiert wird, wobei die Erzeugung des Lautsprechersignals anhand des äußeren akustischen Signals erfolgt.

Analog zum inneren akustischen Signal, kann auch das äußere akustische Signal wird an die Steuereinheit der Audioeinheit übertragen werden, welche das innere akustische Signal verarbeitet und den zumindest einen Lautsprechersignals zur Erzeugung des Lautsprechersignals ansteuert. Damit können beispielsweise noch besser mittels Luftleitung übertragene Geräusche unterdrückt werden.

Zudem ist denkbar, dass das äußere akustische Signal einen Sprachbefehl des Patienten umfasst. Dabei kann durch den Sprachbefehl beispielsweise eine Stärke einer Geräuschunterdrückung und/oder eine Hintergrundmusik eingestellt werden. Zudem kann anhand des akustischen Signals durch die Audioeinheit ein Unbehagen und/oder eine Panikattacke des Patienten erkannt werden, worauf eine Warnung an einen Bediener der Magnetresonanzanlage ausgegeben werden kann.

Eine weitere Ausführungsform sieht vor, dass eine Richtcharakteristik zumindest eines, insbesondere äußeren, Mikrofons anhand einer Aktivität der Gradientenspuleneinheit eingestellt wird. Um die Richtcharakteristik des zumindest einen Mikrofons verändern zu können, weist das zumindest eine Mikrofon - wie bereits erläutert - vorzugsweise ein Mikrofonarray auf.

Beispielsweise kann in einer Ruhephase der Gradientenspuleneinheit, insbesondere in einem Sprachkommunikationsmodus, in dem die Gradientenspuleneinheit keine Gradientenpulse appliziert, d.h. durch die Gradientenspulen der Gradientenspuleneinheit fließt kein Gradientenstrom und/oder durch die Gradientenspuleneinheit werden keine Magnetfeldgradienten erzeugt, die Richtcharakteristik des zumindest einen Mikrofons so eingestellt werden, dass es in Richtung des Sprechorgans des Patienten eine erhöhte Empfindlichkeit aufweist.

Bevorzugt wird in einer Betriebsphase der Gradientenspuleneinheit, insbesondere in einem Geräuschunterdrückungsmodus, die Richtcharakteristik des zumindest einen Mikrofons mit der Aktivität der Gradientenspuleneinheit synchronisiert. Dazu kann beispielsweise der Steuereinheit der Audioeinheit eine MR-Sequenzinformation, beispielsweise zumindest ein Teil einer MR-Pulssequenz, die in der Betriebsphase der Gradientenspuleneinheit durchgeführt wird, bereitgestellt werden. Die Steuereinheit kann ferner die Richtcharakteristik des zumindest einen Mikrofons einstellen Insbesondere kann die Audioeinheit eine maximale räumliche Empfindlichkeit eines Mikrofonarrays in eine gewünschte Richtung steuern, beispielsweise durch Verstärkung und/oder Abschwächung ausgewählter Mikrofonelemente des Mikrofonarrays. Zudem kann das Mikrofonarray so eingestellt werden, dass seine Empfindlichkeit in einer anderen Richtung ausgelöscht wird. Es ist auch denkbar, bei der Einstellung der Richtcharakteristik eine tatsächliche Lage des Kopfes des Patienten im Patientenaufnahmebereich berücksichtigt wird, z. B. in einer Bauchlage, einer Rückenlage oder einer Seitenlage des Patienten.

Vorzugsweise kann die Richtcharakteristik des zumindest einen Mikrofons zwischen zumindest zwei unterschiedlichen Richtcharakteristiken verändert werden, wobei eine erste Richtcharakteristik in einem Stimmaufnahmemodus eingestellt wird und eine zweite Richtcharakteristik in einem Geräuschaufnahmemodus eingestellt wird.

Der Stimmaufnahmemodus wird vorzugsweise dann eingestellt, wenn die Gradientenspuleneinheit inaktiv ist, d.h. es werden keine Gradientenpulse appliziert. Die erste Richtcharakteristik weist vorzugsweise in Richtung des Sprechorgans des Patienten eine erhöhte Empfindlichkeit auf. Damit kann die Stimme des Patienten besser, insbesondere mit einem höheren Signal-Rausch-Verhältnis, mit dem zumindest einen Mikrofon aufgenommen werden.

Der Geräuschaufnahmemodus wird vorzugsweise dann eingestellt, wenn die Gradientenspuleneinheit inaktiv ist, d. h. wenn keine Gradientenpulse appliziert werden. Die zweite Richtcharakteristik weist vorzugsweise eine zur ersten Richtcharakteristik verschiedene Richtcharakteristik auf. Damit können Umgebungsgeräusche, insbesondere verursacht durch die Magnetresonanzanlage, mit dem zumindest einen Mikrofon besser aufgenommen werden.

Vorzugsweise wird der Betrieb der Audioeinheit mit einer MR-Sequenzinformation synchronisiert, z. B. indem die MR-Pulssequenz zumindest teilweise der Audioeinheit bereitgestellt wird. Insbesondere kann dabei die Periodizität, beispielsweise eine Repetitionsrate TR, und/oder das Timing der Gradientenpulse, welche die Geräusche im Wesentlichen kennzeichnen, die durch die Magnetresonanzanlage verursacht werden, von der Magnetresonanzanlage auf die Audioeinheit übertragen werden.

Insbesondere wird vorgeschlagen, dass synchron zu einer MR-Pulssequenz durch den zumindest einen Lautsprecher der Audioeinheit akustische Signale, insbesondere Präventivsignale, ausgegeben werden. Diese akustischen Signale können beispielsweise Musik umfassen. Vorteilhafterweise umfasst die Audioeinheit zur Ausgabe der Präventivsignale zumindest einen Lautsprecher zur Erzeugung der Präventivsignale, eine Steuereinheit zur Ansteuerung des Lautsprechers und eine drahtlose Übertragungseinheit zur Übermittlung etwaiger Synchronisierungssignale, insbesondere von MR-Sequenzinformationen, an die Steuereinheit. Zur Ausgabe eines Präventivsignals ist es nicht notwendig, dass die Audioeinheit Sensoren, wie eine Vibrationssensoreinheit und/oder ein oder mehrere Mikrofone, umfasst.

Unter einem Präventivsignal kann ein akustisches Signal verstanden werden, das in einem Zeitraum vor einer prognostizierten mechanischen Vibration, die durch die Magnetresonanzanlage, insbesondere der Gradientenspuleneinheit der Magnetresonanzanlage verursacht wird, ausgegeben wird. Vorzugsweise ist die Steuereinheit der Audioeinheit ausgebildet, den zumindest einen Lautsprecher zur Ausgabe des Präventivsignals anzusteuern. Vorzugsweise wird die Lautstärke des Präventivsignals kontinuierlich erhöht, d.h. die Lautstärke schwillt an, bevor die mechanische Vibration erzeugt wird. Die Prognose der mechanischen Vibration kann anhand einer Messung einer Variation des durch die Magnetresonanzanlage verursachten Geräuschs erfolgen. Durch die ansteigende Lautstärke kann der Patient effektiv abgelenkt werden, so dass unabsichtliche Bewegungen des Patienten vermieden werden können. Zudem kann dadurch das Gehör des Patienten geschützt werden, indem präventiv biologische Schutzmechanismen des Gehörs, insbesondere des Innenohrs aktiviert werden. Diese Aktivierung kann somit mit deutlich geringeren Lautstärken erreicht werden verglichen mit der Lautstärke, die durch die Gradientenspuleneinheit erzeugt wird.

Ferner wird vorgeschlagen, dass die Magnetresonanzanlage eine MR-Sequenzinformation an die Audioeinheit überträgt, wobei die durch die Sensoren der Audioeinheit erfassten Signale mit Hilfe der Sequenzinformationen separiert werden.

Beispielsweise können sich wiederholende Phasen, in denen die Gradientenspuleneinheit inaktiv und dann wieder aktiv ist, verwendet werden, um mittels eines Mustererkennungsverfahrens künftige Geräuschentwicklungen vorauszusagen. Diese Informationen können verwendet werden, um die Geräuschunterdrückung und/oder die Separierung zwischen Geräusch- und Stimmsignalen weiter zu verbessern.

Ferner wird vorgeschlagen, dass durch eine Vibrationssensoreinheit ein Signal detektiert wird, anhand dessen eine Bewegung und/oder Position eines Patientenkopfs ermittelt wird. Anhand der ermittelten Bewegung und/oder Position des Patientenkopfs kann eine MR-Messung korrigiert werden.

Beispielsweise können mittels eines Mehrachsen-Beschleunigungssensor Signale aufgenommen werden und drahtlos an die Magnetresonanzanlage übertragen werden. Diese Informationen können weiterverarbeitet werden, um Änderungen der Kopfposition während einer MR-Messung festzustellen und die MR-Messung, insbesondere die Magnetresonanzmessdaten, gegebenenfalls um die Änderungen der Kopfposition zur korrigieren.

Ferner kann die ermittelte Bewegung und/oder Position verwendet werden, um Gegenmaßnahmen gegen die Vibrationen des Kopfes vorzunehmen, die durch den Patienten als Lärm wahrgenommen werden. Dazu kann beispielsweise anhand von Signalen eines Mehrachsen-Beschleunigungssensor eine relative Position des Patientenkopfs zur Oberfläche des Patiententisches, auf dem der Patient liegt, ermittelt werden.

Zudem wird ein Computerprogrammprodukt vorgeschlagen, das ein Programm umfasst und direkt in einen Speicher einer programmierbaren Recheneinheit einer Steuereinheit einer Audioeinheit ladbar ist und Programmmittel, z.B. Bibliotheken und Hilfsfunktionen, aufweist, um ein Verfahren zum Betrieb einer Audioeinheit auszuführen, wenn das Computerprogrammprodukt in der Steuereinheit ausgeführt wird. Das Computerprogrammprodukt kann dabei eine Software mit einem Quellcode, der noch kompiliert und gebunden oder der nur interpretiert werden muss, oder einen ausführbaren Softwarecode umfassen, der zur Ausführung nur noch in die Steuereinheit zu laden ist. Durch das Computerprogrammprodukt kann das Verfahren zum Betrieb der Audioeinheit schnell, identisch wiederholbar und robust ausgeführt werden. Das Computerprogrammprodukt ist so konfiguriert, dass es mittels der Steuereinheit die erfindungsgemäßen Verfahrensschritte ausführen kann. Die Steuereinheit muss dabei jeweils die Voraussetzungen wie beispielsweise einen entsprechenden Arbeitsspeicher oder eine entsprechende Logikeinheit aufweisen, so dass die jeweiligen Verfahrensschritte effizient ausgeführt werden können.

Weitere Vorteile, Merkmale und Einzelheiten der Erfindung ergeben sich aus den im Folgenden beschriebenen Ausführungsbeispielen sowie anhand der Zeichnungen. Einander entsprechende Teile sind in allen Figuren mit den gleichen Bezugszeichen versehen.

Es zeigen:
- Fig. 1: eine schematische Darstellung eines Magnetresonanzsystems mit einer Magnetresonanzanlage und einer Audioeinheit,
- Fig. 2: eine Illustration verschiedener Übertragungswege von einem Patienten akustisch wahrnehmbarer Signale,
- Fig. 3: eine schematische Darstellung einer Audioeinheit,
- Fig. 4: eine schematische Darstellung einer Kommunikation zwischen einem Audiosystem und einer Magnetresonanzanlage,
- Fig. 5: eine schematische Darstellung einer Richtcharakteristik eines Mikrofonarrays,
- Fig. 6: eine Blockbild eines Verfahrens zum Betrieb eines Audiosystems.

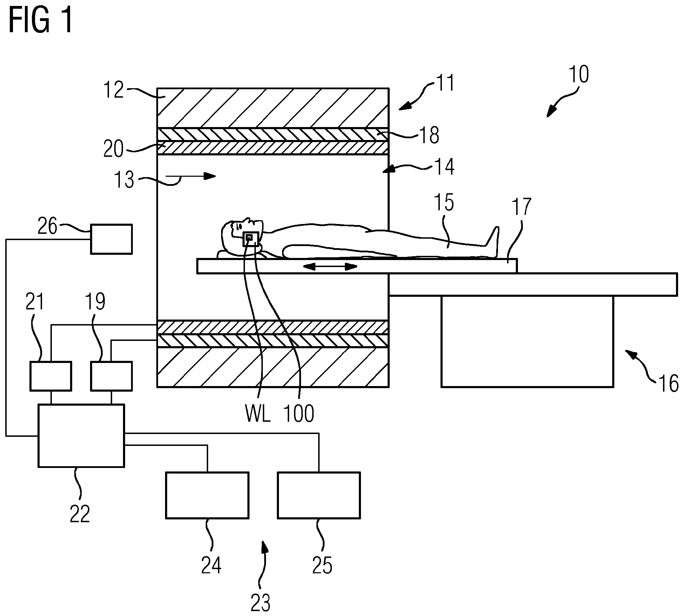

In Fig. 1 ist Magnetresonanzsystem schematisch dargestellt, das eine Magnetresonanzanlage 10 und eine Audioeinheit 100 umfasst. Die Magnetresonanzanlage 10 umfasst eine Magneteinheit 11, die einen Hauptmagneten 12 zu einem Erzeugen eines starken und insbesondere zeitlich konstanten Hauptmagnetfelds 13 aufweist. Zudem umfasst die Magnetresonanzanlage 10 einen Patientenaufnahmebereich 14 zu einer Aufnahme eines Patienten 15. Der Patientenaufnahmebereich 14 im vorliegenden Ausführungsbeispiel ist zylinderförmig ausgebildet und in einer Umfangsrichtung von einer Abdeckung der Magneteinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzanlage 10 in den Patientenaufnahmebereich 14 geschoben werden. Die Patientenlagerungsvorrichtung 16 weist hierzu einen innerhalb des Patientenaufnahmebereichs 14 bewegbar ausgestalteten Patiententisch 17 auf.

Die Magneteinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 weist üblicherweise mehrere Gradientenspulen auf, die hier nicht gesondert dargestellt sind. Durch die Gradientenspulen fließen Gradientenströme, welche die Magnetfeldgradienten verursachen. Da die Gradientenströme üblicherweise gepulst durch die Gradientenspulen fließen, spricht man auch von Gradientenpulsen. Die Gradientenspuleneinheit 18 wird mittels einer Gradientensteuereinheit 19 der Magnetresonanzanlage 10 gesteuert.

Die Magneteinheit 11 umfasst weiterhin eine Hochfrequenzantenneneinheit 20, welche im vorliegenden Ausführungsbeispiel als fest in die Magnetresonanzanlage 10 integrierte Körperspule ausgebildet ist. Die Hochfrequenzantenneneinheit 20 ist zu einer Anregung von Atomkernen, die sich in dem von dem Hauptmagneten 12 erzeugten Hauptmagnetfeld 13 einstellt, ausgelegt. Die Hochfrequenzantenneneinheit 20 wird von einer Hochfrequenzantennensteuereinheit 21 der Magnetresonanzanlage 10 gesteuert und strahlt Hochfrequenzpulse in einen Untersuchungsraum ein, der im Wesentlichen von einem Patientenaufnahmebereich 14 der Magnetresonanzanlage 10 gebildet ist. Die Hochfrequenzantenneneinheit 20 ist weiterhin zum Empfang von Magnetresonanzsignalen ausgebildet.

Zu einer Steuerung des Hauptmagneten 12, der Gradientensteuereinheit 19 und zur Steuerung der Hochfrequenzantennensteuereinheit 21 weist die Magnetresonanzanlage 10 eine Anlagensteuereinheit 22 auf. Die Anlagensteuereinheit 22 steuert zentral die Magnetresonanzanlage 10, wie beispielsweise das Durchführen einer vorbestimmten bildgebenden Gradientenechosequenz. Zudem umfasst die Anlagensteuereinheit 22 eine nicht näher dargestellte Auswerteeinheit zu einer Auswertung von medizinischen Bilddaten, die während der Magnetresonanzuntersuchung erfasst werden. Des Weiteren umfasst die Magnetresonanzanlage 10 eine Benutzerschnittstelle 23, die mit der Anlagensteuereinheit 22 verbunden ist. Informationen, etwa Steuerinformationen wie beispielsweise Bildgebungsparameter, sowie rekonstruierte Magnetresonanzbilder können durch eine Ausgabeeinheit 24, beispielsweise auf einem Monitor oder durch einen Lautsprecher, der Benutzerschnittstelle 23 für ein medizinisches Bedienpersonal ausgegeben werden. Weiterhin weist die Benutzerschnittstelle 23 eine Eingabeeinheit 25 auf, mittels der Informationen und/oder Parameter während eines Messvorgangs von dem medizinischen Bedienpersonal eingegeben werden können.

Die Audioeinheit umfasst eine erste drahtlose Übertragungseinheit WL, die ausgebildet ist, elektromagnetische Signale zu detektieren und zu senden. Auch die Magnetresonanzanlage 10 umfasst eine weitere drahtlose Übertragungseinheit 26, die mit der Anlagensteuereinheit 22 verbunden und ausgebildet ist, elektromagnetische Signale zu detektieren und zu senden. Über die drahtlose Übertragungseinheiten WL, 26 können die Magnetresonanzanlage 10 und die Audioeinheit miteinander kommunizieren, d.h. es können beispielsweise Informationen, insbesondere Signale, ausgetauscht werden.

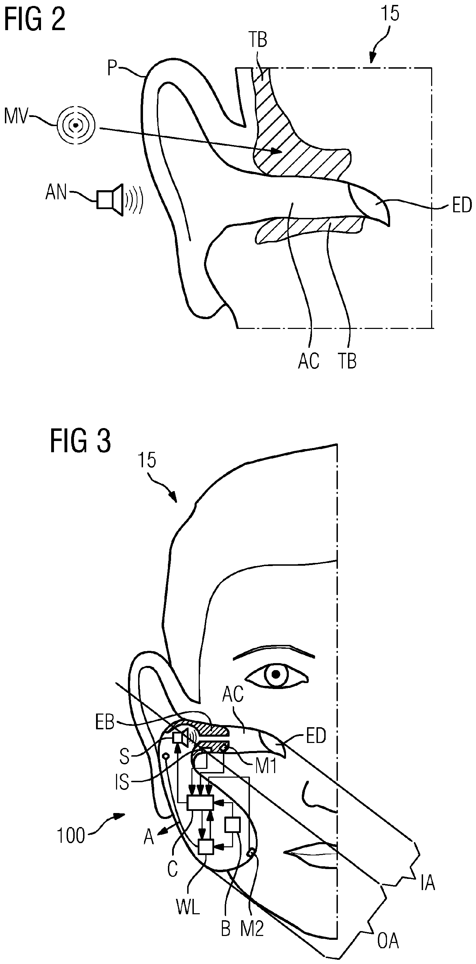

In Fig. 2 werden verschiedene Übertragungswege von Signalen illustriert, die durch den Patienten 15 akustische wahrgenommen werden können. Einerseits können Schalldruckwellen AN über die Luft übermittelt werden. Vibrationen, die durch die Gradientenspulen verursacht werden, rufen Vibrationen der Abdeckung der Magneteinheit 11 hervor, welche wiederum in Schalldruckwellen AN innerhalb des Patientenaufnahmebereichs 14 umgewandelt werden. Diese Schalldruckwellen AN breiten sich per Luftleitung durch die Ohrmuschel P über den, insbesondere äußeren, Gehörgang AC bis zum Trommelfell ED aus, welches dadurch zum Schwingen angeregt wird. Über die hier nicht dargestellte Gehörknöchelchen des Mittelohrs werden die Schwingungen an die Hörschnecke weitergeleitet und von dieser in elektrische Signale umgesetzt, die an das Gehirn des Patienten 15 gesendet werden.

Ein weiterer Übertragungsweg erfolgt über Knochenleitung. Vibrationen der Gradientenspulen verursachen mechanische Vibrationen MV des Patiententisches 17, auf dem der Patient 15 gelagert ist. Über die Oberfläche des Patiententisches und dann weiter über den Schädel und/oder das Schläfenbein TB und/oder die Wand des Gehörgangs AC etc. wird schließlich die Hörschnecke stimuliert. Diese Stimulation ist unabhängig von den Luftdruckschwankungen innerhalb des Gehörgangs AC.

In Fig. 3 wird eine Audioeinheit 100 zur Unterdrückung einer Wahrnehmung eines durch eine Magnetresonanzanlage 10 verursachten Geräuschs dargestellt, die zumindest einen Übertragungsweg, vorteilhafterweise aber beide Übertragungswege, also die Luftleitung und die Knochenleitung berücksichtigt.

Vorzugsweise sind die elektrischer Drähte und/oder Antennen und/oder Leitungen innerhalb der Audioeinheit deutlich kleiner als die Wellenlänge der Hochfrequenz-Strahlung, die bei einer MR-Messung üblicherweise verwendet wird. Dies ist üblicherweise der Fall, wenn die Audioeinheit kleiner als 5 cm, idealerweise kleiner als 3 cm ist.

Die Audioeinheit 100 umfasst eine Vibrationssensoreinheit IS, eine Steuereinheit C und einen Lautsprecher S. Die Vibrationssensoreinheit IS ist ausgebildet, mittels Knochenleitung übertragene Signale MV zu detektieren und an die Steuereinheit C zu übertragen. Die Steuereinheit C kann hier nicht dargestellte analoge und/oder digitale Verarbeitungseinheiten umfassen, um die an sie übertragenen Signale zu verarbeiten. Die Steuereinheit C ist ausgebildet, anhand der übertragenen Signale den Lautsprecher S anzusteuern.

Optional umfasst die Audioeinheit eine erste drahtlose Übertragungseinheit WL, die ausgebildet ist, elektromagnetische Signale zu detektieren und an die Steuereinheit C übertragen und/oder von der Steuereinheit C empfangene Signale als elektromagnetische Signale auszusenden. Die drahtlose Übertragungseinheit WL umfasst eine Antenne A, mit der die elektromagnetischen Signalen empfangen und ausgesendet werden können.

Die Audioeinheit 100 umfasst ferner einen Energiespeicher B, beispielsweise eine Batterie, die mit der Steuereinheit C und der drahtlosen Übertragungseinheit WL verbunden ist und diese mit elektrischer Energie versorgen kann.

Die Vibrationssensoreinheit IS umfasst vorzugsweise zumindest einen Inertialsensor und/oder zumindest einen Beschleunigungssensor und/oder zumindest einen Drehratensensor. In einer Ausführungsform als Mehrachsen-Beschleunigungssensor kann die Vibrationssensoreinheit IS Signale erfassen, die Rückschlüsse auf die Quelle des Signals zulassen.

Die Vibrationssensoreinheit IS ist bevorzugt ausgebildet, mechanische Schwingungen MV mit Frequenzen zwischen 15 Hz und 20 kHz Hz, insbesondere zwischen 15 Hz und 1 kHz, zu detektieren, da diese üblicherweise besonders stark durch den Betrieb der Gradientenspuleneinheit 18 erzeugt werden.

Die Audioeinheit 100 umfasst einen ersten Abschnitt IA, der eine Form aufweist, die geeignet ist, den ersten Abschnitt in den Gehörgang AC des Patienten 15 einzuführen. Dabei ist die Vibrationssensoreinheit IS im ersten Abschnitt IA der Audioeinheit 100 angeordnet. Insbesondere ist die Vibrationssensoreinheit IS direkt an der Wand des Gehörgangs AC angeordnet, so dass die mechanischen Vibrationen MV besonders effektiv detektiert werden können. Es ist aber auch denkbar, dass eine schallleitende Zwischenschicht zwischen der Vibrationssensoreinheit IS und der Wand des Gehörgangs AC angeordnet ist.

Der erste Abschnitt kann insbesondere ein Earbud EB umfassen und/oder eine Form eines Im-Ohr-Kopfhörers (engl. In-ear headphones) und/oder eines Earbud-Ohrhörer aufweisen. Dies ermöglicht eine stabile Befestigung der Audioeinheit 100 im Gehörgang AC des Patienten 15.

Optional umfasst die Audioeinheit ein oder mehrere Mikrofone, die ausgebildet sind, mittels Luftleitung übertragene Signale AN zu detektieren und an die Steuereinheit C zu übertragen. In diesem Beispiel umfasst die Audioeinheit ein inneres Mikrofon M1 und ein äußeres Mikrofon M2. Das innere Mikrofon ist im ersten Abschnitt IA angeordnet. Somit können mit dem inneren Mikrofon M1 die per Luftleitung übertragenen Signale im Gehörgang AC detektiert werden.

Neben dem ersten Abschnitt IA, weist die Audioeinheit 100 einen zweiten Abschnitt OA auf, der bei Einführung des ersten Abschnitts IA in den Gehörgang AC außerhalb des Gehörgangs AC angeordnet ist. Das äußere Mikrofon M2 ist hier im zweiten Abschnitt OA der Audioeinheit 100 angeordnet.

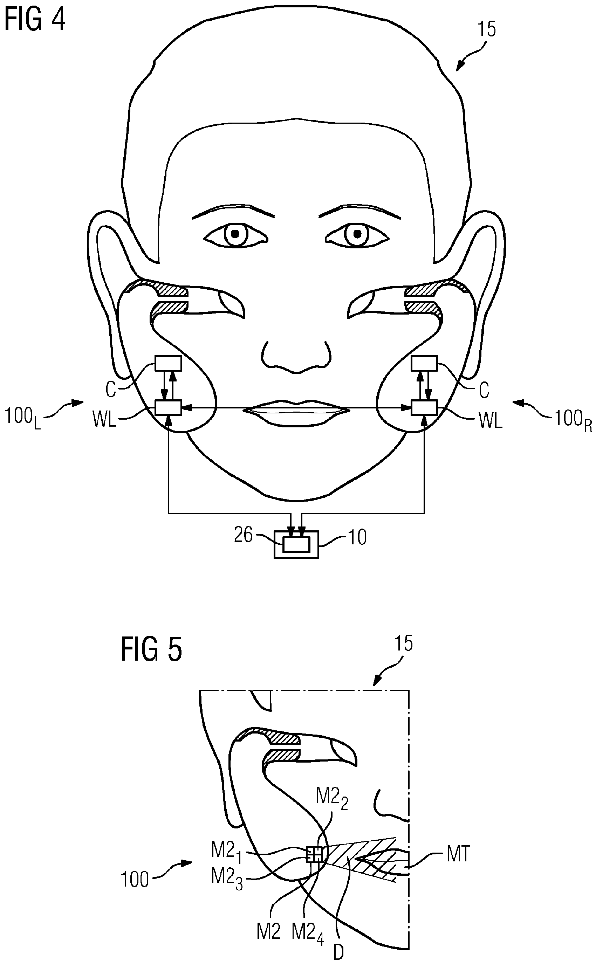

Das äußere Mikrofon M2 umfasst vorzugsweise ein Mikrofonarray, welches beispielhaft in Fig. 5 dargestellt ist. Das Mikrofonarray umfasst in diesem Beispiel vier Mikrofonelemente M2₁, M2₂, M2₃, M2₄, wobei ein Mikrofonarray auch weniger, aber vor allem auch deutlich mehr Mikrofonelemente aufweisen kann. Durch eine geeigneten Betrieb des Mikrofonarrays, etwa durch die Steuereinheit C, kann die Richtcharakteristik D des beeinflusst werden. Hier ist die Richtcharakteristik D beispielsweise so eingestellt, dass sie in Richtung des Mundes MT und/oder der Stimme des Patienten eine erhöhte relative Empfindlichkeit aufweist. Somit können gesprochene Signale des Patienten 15 mit einem erhöhten Signal-Rausch-Verhältnis durch das äußere Mikrofon M2 erfasst werden.

In Fig. 4 ist ein Audiosystem mit einer ersten Audioeinheit 100_{L} und einer zweiten Audioeinheit 100_{R}. Beide Audioeinheiten 100_{L}, 100_{R} weisen jeweils eine drahtlose Übertragungseinheit WL auf, mit denen sie miteinander kommunizieren können.

Sie können jedoch nicht nur miteinander kommunizieren, sondern auch mit der Magnetresonanzanlage 10, die ebenfalls eine drahtlose Übertragungseinheit 26 umfasst. Zusammen mit den Audioeinheiten 100_{L}, 100_{R} bildet die Magnetresonanzanlage 10 hier ein Magnetresonanzsystem.

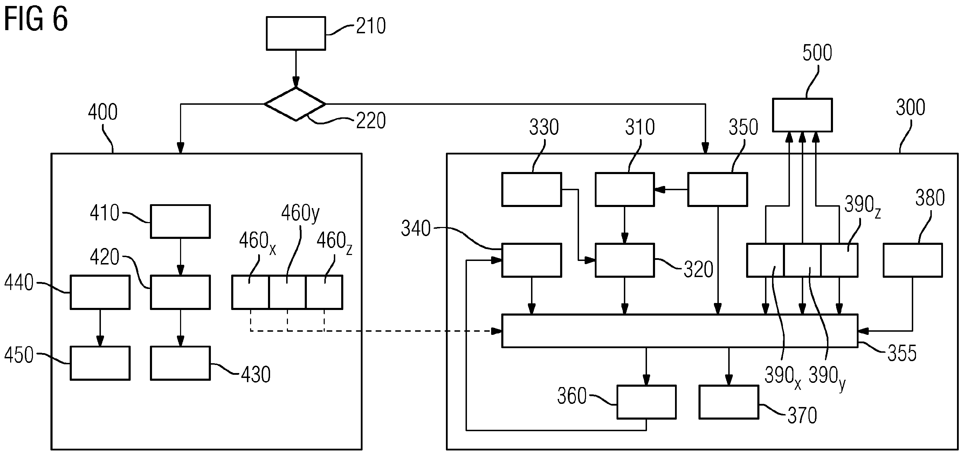

In Fig. 6 ist ein Verfahren zum Betrieb einer Audioeinheit 100 dargestellt. In 210 wird ein Betriebszustand der Gradientenspuleneinheit 18 der Magnetresonanzanlage 10 bereitgestellt. Davon abhängig wird in 220 entschieden, wie wird Audioeinheit 100 betrieben wird.

In einer Ruhephase der Gradientenspuleneinheit 18 der Magnetresonanzanlage 10 wird die Audioeinheit 100 in einem Sprachkommunikationsmodus 400 betrieben. Dabei wird in 420 ein akustisches Signal durch das äußere Mikrofon M2 der Audioeinheit 100 detektiert, das in 430 durch eine drahtlose Übertragungseinheit WL der Audioeinheit 100 an eine drahtlose Übertragungseinheit 26 der Magnetresonanzanlage 10 übertragen und durch die Ausgabeeinheit 24 ausgegeben wird. Vorher kann optional in 410 die Richtcharakteristik optimiert für den Sprachkommunikationsmodus 400, in dem die Gradientenspuleneinheit nicht aktiv ist, eingestellt werden.

Ferner wird in 440 ein Signal durch die drahtlose Übertragungseinheit 26 der Magnetresonanzanlage 10 an die drahtlose Übertragungseinheit WL der Audioeinheit 100 übertragen und in 450 durch den Lautsprecher S der Audioeinheit ausgegeben.

In einer Betriebsphase der Gradientenspuleneinheit 18 der Magnetresonanzanlage 10 wird in 390x, 390y, 390z durch die Vibrationssensoreinheit IS der Audioeinheit 100 ein mittels Knochenleitung übertragenes Knochenleitungssignal MV detektiert und an die Steuereinheit C übertragen. Insbesondere wird in 390x eine x-Komponente, in 390y eine y-Komponente und in 390z eine z-Komponente des Knochenleitungssignal MV detektiert. Das Knochenleitungssignal umfasst also mehrere richtungsabhängige Knochenleitungssignale. In 355 werden die an die Steuereinheit C übertragenen Signale, insbesondere das Knochenleitungssignal ausgewertet.

Vorzugsweise wird in 355 das Knochenleitungssignal in Abhängigkeit der mehreren richtungsabhängigen Knochenleitungssignale in zumindest zwei Anteile separiert. Dazu werden Knochenleitungssignale verwendet, die in 460x, 460y, 460z während einer Ruhephase der Gradientenspuleneinheit 18 der Magnetresonanzanlage 10 durch die Stimme eines Patienten 15 erzeugt und durch die Vibrationssensoreinheit IS detektiert wurden.

Optional kann in 380 eine Zuordnungstabelle der Steuereinheit C bereitgestellt werden. Die Zuordnungstabelle kann insbesondere empirisch ermittelte Vibrationsmuster umfassen, denen Kompensationsmuster zugeordnet sind. Diese Informationen können in 355 durch die Steuereinheit C verarbeitet werden. Insbesondere können die Kompensationsmuster verwendet werden, um in 360 ein geeignetes Lautsprechersignal zu erzeugen, das Knochenleitungssignal MV kompensiert, d. h. reduziert oder sogar auslöscht.

Optional wird in 340 durch das innere Mikrofon M1 der Audioeinheit 100 ein inneres akustisches Signal innerhalb des Gehörgangs AC detektiert. Anhand des inneren akustischen Signals erfolgt die Erzeugung des Lautsprechersignals, d.h. die Steuereinheit C wertet in 355 das innere akustische Signal, neben etwaigen anderen Signalen, aus und steuert den Lautsprecher S an, der in 360 ein Lautsprechersignal erzeugt.

Optional wird in 340 durch das äußere Mikrofon M2 der Audioeinheit 100 ein äußeres akustisches Signal außerhalb des Gehörgangs AC detektiert. Anhand des äußeres akustischen Signals, erfolgt die Erzeugung des Lautsprechersignals, d.h. die Steuereinheit C wertet in 355 das innere akustische Signal, neben etwaigen anderen Signalen, aus und steuert den Lautsprecher S an, der in 360 ein Lautsprechersignal erzeugt. Das äußere akustische Signal kann auch einen Sprachbefehl umfassen, der in 330 ausgegeben wird

Vorzugsweise wird der Betrieb der Audioeinheit 100 mit einer MR-Sequenzinformation synchronisiert, die in 350 bereitgestellt wird. Mit Hilfe der MR-Sequenzinformation kann beispielsweise in 310 die Richtcharakteristik des äußeren Mikrofons M2 eingestellt werden.

Ferner kann in einem Zeitraum vor einer prognostizierten mechanischen Vibration MV, die durch die Gradientenspuleneinheit 18 der Magnetresonanzanlage 10 verursacht wird, ein Präventivsignal durch den Lautsprecher S ausgegeben werden.

Es wird abschließend noch einmal darauf hingewiesen, dass es sich bei den vorhergehend detailliert beschriebenen Verfahren sowie bei der dargestellten Audioeinheit, dem dargestellten Audiosystem und dem dargestellten Magnetresonanzsystem lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließt der Begriff "Einheit" nicht aus, dass die betreffenden Komponenten aus mehreren zusammenwirkenden Teil-Komponenten bestehen, die gegebenenfalls auch räumlich verteilt sein können.

## Patentansprüche

1. Audioeinheit (100) zur Unterdrückung einer Wahrnehmung eines durch eine Magnetresonanzanlage (10) verursachten Geräuschs, wobei die Audioeinheit (100) zumindest einen Lautsprecher (S) umfasst, sowie eine Vibrationssensoreinheit (IS), die ausgebildet ist, mittels Knochenleitung übertragene Signale zu detektieren,
**dadurch gekennzeichnet, dass** die Audioeinheit über eine Steuereinheit (C) verfügt und die Vibrationssensoreinheit ausgebildet ist, die mittels Knochenleitung übertragenen Signale an die Steuereinheit (C) zu übertragen, wobei die Steuereinheit (C) ausgebildet ist, anhand der mittels Knochenleitung übertragenen Signale ein destruktiv interferierendes Signal zu ermitteln und über den Lautsprecher (S) auszugeben.

2. Audioeinheit (100) nach Anspruch 1,
wobei die Vibrationssensoreinheit (IS) zumindest einen Inertialsensor und/oder zumindest einen Beschleunigungssensor und/oder zumindest einen Drehratensensor umfasst.

3. Audioeinheit (100) nach einem der Ansprüche 1 bis 2,
wobei die Vibrationssensoreinheit (IS) zumindest einen Mehrachsen-Beschleunigungssensor umfasst.

4. Audioeinheit (100) nach einem der vorangehenden Ansprüche,
wobei die Vibrationssensoreinheit (IS) ausgebildet ist, mechanische Schwingungen mit Frequenzen zwischen 15 Hz und 20 kHz zu detektieren.

5. Audioeinheit (100) nach einem der vorangehenden Ansprüche,
wobei die Audioeinheit (100) einen ersten Abschnitt umfasst, der eine Form aufweist, die geeignet ist, den ersten Abschnitt in einen Gehörgang (AC) eines Patienten (15) einzuführen,
wobei die Vibrationssensoreinheit (IS) im ersten Abschnitt der Audioeinheit (100) angeordnet ist.

6. Audioeinheit (100) nach einem der vorangehenden Ansprüche,
wobei die Audioeinheit (100) kleiner als 5 cm ist.

7. Audioeinheit (100) nach einem der vorangehenden Ansprüche,
wobei die Audioeinheit (100) zumindest ein Mikrofon (M1, M2) umfasst, das ausgebildet ist, mittels Luftleitung übertragene Signale zu detektieren und an die Steuereinheit (C) zu übertragen.

8. Audioeinheit (100) nach einem der Ansprüche 1 bis 4,
wobei die Audioeinheit (100) einen ersten Abschnitt umfasst, der eine Form aufweist, die geeignet ist, den ersten Abschnitt in einen Gehörgang (AC) einzuführen,
wobei die Audioeinheit (100) ein inneres Mikrofon (M1) umfasst, das im ersten Abschnitt der Audioeinheit (100) angeordnet ist,
wobei die Audioeinheit (100) einen zweiten Abschnitt umfasst, der bei
Einführung des ersten Abschnitts in den Gehörgang (AC) außerhalb des Gehörgangs (AC) angeordnet ist,
wobei die Audioeinheit (100) ein äußeres Mikrofon (M2) umfasst, das im zweiten Abschnitt der Audioeinheit (100) angeordnet ist,
wobei das innere (M1) und das äußere Mikrofon (M2) ausgebildet sind, mittels Luftleitung übertragene Signale zu detektieren und an die Steuereinheit (C) zu übertragen.

9. Audioeinheit (100) nach Anspruch 8,
wobei das äußere Mikrofon (M2) ein Mikrofonarray umfasst.

10. Audioeinheit (100) nach einem der vorangehenden Ansprüche,
wobei die Audioeinheit (100) eine erste drahtlose Übertragungseinheit umfasst, die ausgebildet ist, elektromagnetische Signale zu detektieren und an die Steuereinheit (C) übertragen und/oder von der Steuereinheit (C) empfangene Signale als elektromagnetische Signale auszusenden.

11. Audiosystem mit einer ersten Audioeinheit (100_{L}) nach einem der Ansprüche 1 bis 10 und einer zweiten Audioeinheit (100_{R}) nach einem der Ansprüche 1 bis 10.

12. Audiosystem nach Anspruch 11,
wobei die erste Audioeinheit (100_{L}) und die zweite Audioeinheit (100_{R}) ausgebildet sind, miteinander zu kommunizieren.

13. Magnetresonanzanlage (10) mit einem Audiosystem nach Anspruch 11 oder 12 und/oder einer Audioeinheit (100) nach einem der Ansprüche 1 bis 10.

14. Magnetresonanzanlage nach Anspruch 13,
wobei die Magnetresonanzanlage (10), das Audiosystem und/oder die Audioeinheit (100) ausgebildet sind, miteinander zu kommunizieren.

15. Verfahren zum Betrieb einer Audioeinheit (100),
wobei in einer Betriebsphase einer Gradientenspuleneinheit (18) einer Magnetresonanzanlage (10) durch eine Vibrationssensoreinheit (IS) der Audioeinheit (100) ein mittels Knochenleitung übertragenes Knochenleitungssignal detektiert wird, **dadurch gekennzeichnet,**
**dass** anhand des Knochenleitungssignals durch zumindest einen Lautsprecher (S) der Audioeinheit (100) ein destruktiv interferierendes Lautsprechersignal erzeugt wird.

16. Verfahren nach Anspruch 15,
wobei das Knochenleitungssignal mehrere richtungsabhängige Knochenleitungssignale umfassen,
wobei das Knochenleitungssignal in Abhängigkeit der mehreren richtungsabhängigen Knochenleitungssignale in zumindest einen Sprachanteil und einen Geräuschanteil separiert wird.

17. Verfahren nach Anspruch 16,
wobei in einer Ruhephase der Gradientenspuleneinheit (18) der Magnetresonanzanlage (10) durch die Stimme eines Patienten (15) Knochenleitungssignale erzeugt werden, die durch die Vibrationssensoreinheit (IS) detektiert werden und anhand derer in einer Betriebsphase des Gradientenspuleneinheit (18) der Magnetresonanzanlage (10) das Knochenleitungssignal in den zumindest einen Sprachanteil und einen Geräuschanteil separiert werden.

18. Verfahren nach einem der Ansprüche 15 bis 17,
wobei in einer Betriebsphase der Gradientenspuleneinheit (18) durch ein Mikrofon (M1) der Audioeinheit (100) ein inneres akustisches Signal innerhalb eines Gehörgangs (AC) detektiert wird,
wobei die Erzeugung des Lautsprechersignals anhand des inneren akustischen Signals erfolgt.

19. Verfahren nach einem der Ansprüche 15 bis 18,
wobei in einer Betriebsphase der Gradientenspuleneinheit (18) durch ein Mikrofon (M2) der Audioeinheit (100) ein äußeres akustisches Signal außerhalb eines Gehörgangs (AC) detektiert wird,
wobei die Erzeugung des Lautsprechersignals anhand des äußeren akustischen Signals erfolgt.

20. Verfahren nach Anspruch 19,
wobei das äußere akustische Signal einen Sprachbefehl des Patienten (15) umfasst.

21. Verfahren nach einem der Anspruch 15 bis 20,
wobei in einer Ruhephase der Gradientenspuleneinheit (18) der Magnetresonanzanlage (10)
- ein akustisches Signal durch zumindest ein Mikrofon der Audioeinheit (100) detektiert wird, durch eine drahtlose Übertragungseinheit der Audioeinheit (100) an eine drahtlose Übertragungseinheit der Magnetresonanzanlage (10) übertragen und durch eine Ausgabeeinheit ausgegeben wird und/oder
- ein Signal durch die drahtlose Übertragungseinheit der Magnetresonanzanlage (10) an die drahtlose Übertragungseinheit der Audioeinheit (100) übertragen wird und durch einen Lautsprecher (S) der Audioeinheit (100) ausgegeben wird.

22. Verfahren nach einem der Ansprüche 18 bis 21,
wobei eine Richtcharakteristik zumindest eines Mikrofons (M2) anhand einer Aktivität der Gradientenspuleneinheit (18) eingestellt wird.

23. Verfahren nach einem der Ansprüche 15 bis 22,
wobei der Betrieb der Audioeinheit (100) mit einer MR-Sequenzinformation synchronisiert wird.

24. Verfahren nach Anspruch 23,
wobei in einem Zeitraum vor einer prognostizierten mechanischen Vibration, die durch eine Gradientenspuleneinheit (18) einer Magnetresonanzanlage (10) verursacht wird, ein Präventivsignal ausgegeben wird.

25. Verfahren nach einem der Ansprüche 15 bis 24,
wobei durch die Vibrationssensoreinheit (IS) der Audioeinheit (100) ein Signal detektiert wird, anhand dessen eine Bewegung und/oder Position eines Patientenkopfs ermittelt wird.

## Claims

1. Audio unit (100) for reducing an awareness of a noise produced by a magnetic resonance machine (10), wherein the audio unit (100) comprises at least one loudspeaker (S), as well as a vibration sensor unit (IS), which is designed to detect signals transmitted by means of bone conduction,
**characterised in that** the audio unit possesses a control unit (C) and the vibration sensor unit is designed to transmit the signals transmitted by means of bone conduction to the control unit (C),
wherein the control unit (C) is designed, to ascertain a destructively interfering signal on the basis of the signals transmitted by means of bone conduction, and to output said destructively interfering signal via the loudspeaker (S).

2. Audio unit (100) according to claim 1,
wherein the vibration sensor unit (IS) comprises at least one inertial sensor and/or at least one acceleration sensor and/or at least one yaw-rate sensor.

3. Audio unit (100) according to one of claims 1 to 2,
wherein the vibration sensor unit (IS) comprises at least one multi-axis acceleration sensor.

4. Audio unit (100) according to one of the preceding claims,
wherein the vibration sensor unit (IS) is designed to detect mechanical vibrations at frequencies between 15 Hz and 20 kHz.

5. Audio unit (100) according to one of the preceding claims,
wherein the audio unit (100) comprises a first segment, which has a shape that is suitable for inserting the first segment into an auditory canal (AC) of a patient (15),
wherein the vibration sensor unit (IS) is disposed in the first segment of the audio unit (100).

6. Audio unit (100) according to one of the preceding claims,
wherein the audio unit (100) is smaller than 5 cm.

7. Audio unit according to one of the preceding claims,
wherein the audio unit (100) comprises at least one microphone (M1, M2), which is designed to detect signals transmitted by means of air conduction and to transmit said signals to the control unit (C).

8. Audio unit according to one of claims 1 to 4,
wherein the audio unit (100) comprises a first segment, which has a shape that is suitable for inserting the first segment into an auditory canal (AC),
wherein the audio unit (100) comprises an internal microphone (M1), which is disposed in the first segment of the audio unit (100),
wherein the audio unit (100) comprises a second segment, which on insertion of the first segment into the auditory canal (AC) is arranged outside the auditory canal (AC),
wherein the audio unit (100) comprises an external microphone (M2), which is disposed in the second segment of the audio unit (100),
wherein the internal microphone (M1) and the external microphone (M2) are designed to detect signals transmitted by means of air conduction and to transmit said signals to the control unit (C).

9. Audio unit (100) according to claim 8,
wherein the external microphone (M2) comprises a microphone array.

10. Audio unit according to one of the preceding claims,
wherein the audio unit (100) comprises a first wireless transmission unit, which is designed to detect electromagnetic signals and transmit these to the control unit (C), and/or to emit signals received from the control unit (C) as electromagnetic signals.

11. Audio system comprising a first audio unit (100_{L}) according to one of claims 1 to 10 and a second audio unit (100_{R}) according to one of claims 1 to 10.

12. Audio system according to claim 11,
wherein the first audio unit (100_{L}) and the second audio unit (100_{R}) are designed to communicate with each other.

13. Magnetic resonance machine comprising an audio system according to claim 11 or 12 and/or an audio unit (100) according to one of claims 1 to 10.

14. Magnetic resonance machine according to claim 13,
wherein the magnetic resonance machine (10), the audio system and/or the audio unit (100) are designed to communicate with one another.

15. Method for operating an audio unit (100),
wherein during an operating phase of a gradient coil unit (18) of a magnetic resonance machine (10), a vibration sensor unit (IS) of the audio unit (100) detects a bone-conduction signal, which is transmitted by means of bone conduction,
**characterised in that**
at least one loudspeaker (S) of the audio unit (100) generates a destructively interfering loudspeaker signal on the basis of the bone-conduction signal.

16. Method according to claim 15,
wherein the bone-conduction signal comprises a plurality of directional bone-conduction signals,
wherein the bone-conduction signal is separated into at least a voice component and a noise component on the basis of the plurality of directional bone-conduction signals.

17. Method according to claim 16,
wherein during a rest phase of the gradient coil unit (18) of the magnetic resonance machine (10), bone-conduction signals are generated by the voice of a patient (15), which are detected by the vibration sensor unit (IS) and used during an operating phase of the gradient coil unit (18) of the magnetic resonance machine (10) as the basis for separating the bone-conduction signal into the at least one voice component and one noise component.

18. Method according to one of claims 15 to 17,
wherein during an operating phase of the gradient coil unit (18), a microphone (M1) of the audio unit (100) detects an internal acoustic signal inside an auditory canal (AC), wherein the loudspeaker signal is generated on the basis of the internal acoustic signal.

19. Method according to one of claims 15 to 18,
wherein during an operating phase of the gradient coil unit (18), a microphone (M2) of the audio unit (100) detects an external acoustic signal outside an auditory canal (AC), wherein the loudspeaker signal is generated on the basis of the external acoustic signal.

20. Method according to claim 19,
wherein the external acoustic signal includes a voice command of the patient (15).

21. Method according to one of claims 15 to 20,
wherein during a rest phase of the gradient coil unit (18) of the magnetic resonance machine (10)
- at least one microphone of the audio unit (100) detects an acoustic signal, which is transmitted by a wireless transmission unit of the audio unit (100) to a wireless transmission unit of the magnetic resonance machine (10), and is output by an output unit, and/or
- a signal is transmitted by the wireless transmission unit of the magnetic resonance machine (10) to the wireless transmission unit of the audio unit (100), and is output by a loudspeaker (S) of the audio unit (100).

22. Method according to one of claims 18 to 21,
wherein a directivity of at least one microphone (M2) is set on the basis of an activity of the gradient coil unit (18).

23. Method according to one of claims 15 to 22,
wherein the operation of the audio unit (100) is synchronised with MR sequence data.

24. Method according to claim 23,
wherein a preventive signal is output in a time period before a predicted mechanical vibration produced by a gradient coil unit (18) of a magnetic resonance machine (10).

25. Method according to one of claims 15 to 24,
wherein the vibration sensor unit (IS) of the audio unit (100) detects a signal from which a movement and/or position of a patient head is ascertained.

## Revendications

1. Unité (100) audio pour supprimer la perception d'un bruit provoqué par une installation (10) de résonnance magnétique, l'unité (100) audio comprenant au moins un haut-parleur (S), ainsi qu'une unité (IS) de capteur de vibrations, qui est constituée pour détecter des signaux transmis au moyen de la conduction osseuse, **caractérisé en ce que** l'unité audio dispose d'une unité (C) de commande et l'unité de capteur de vibrations est constituée pour transmettre à l'unité (C) de commande les signaux transmis au moyen de la conduction osseuse,
dans laquelle l'unité (C) de commande est constitué pour déterminer, à l'aide des signaux transmis au moyen de la conduction osseuse, un signal d'interférence en destruction et pour le sortir par le haut-parleur (S).

2. Unité (100) audio suivant la revendication 1,
dans laquelle l'unité (IS) de capteur de vibrations comprend au moins un capteur inertiel et/ou au moins un capteur d'accélération et/ou au moins un capteur de vitesse de rotation.

3. Unité (100) audio suivant l'une des revendications 1 à 2,
dans laquelle l'unité (IS) de capteur de vibrations comprend au moins un capteur d'accélération à plusieurs axes.

4. Unité (100) audio suivant l'une des revendications précédentes,
dans laquelle l'unité (IS) de capteur de vibrations est constituée pour détecter des oscillations mécaniques de fréquence comprises entre 15 Hz et 20 kHz.

5. Unité (100) audio suivant l'une des revendications précédentes,
dans laquelle l'unité (100) audio comprend une première partie ayant une forme propre à introduire la première partie dans un conduit (AC) auditif d'un patient (15),
l'unité (IS) de capteur de vibrations étant disposée dans la première partie de l'unité (100) audio.

6. Unité (100) audio suivant l'une des revendications précédente,
dans laquelle l'unité (100) audio est plus petite que 5 cm.

7. Unité (100) audio suivant l'une des revendications précédentes,
dans laquelle l'unité (100) audio comprend au moins un microphone (M1, M2), qui est constitué pour détecter des signaux transmis au moyen de la conduction de l'air et les transmettre à l'unité (C) de commande.

8. Unité (100) audio suivant l'une des revendications 1 à 4,
dans laquelle l'unité (100) audio comprend une première partie, qui a une forme propre à introduire la première partie dans un conduit (AC) auditif,
dans laquelle l'unité (100) audio comprend un microphone (M1) intérieur, qui est disposé dans la première partie de l'unité (100) audio,
dans laquelle l'unité (100) audio comprend une deuxième partie, qui, lors de l'introduction de la première partie dans le conduit (AC) auditif, est disposée à l'extérieur du conduit (AC) auditif,
dans laquelle l'unité (100) audio comprend un microphone (M2) extérieur, qui est disposé dans la deuxième partie de l'unité (100) audio,
dans laquelle le microphone (M1) intérieur et le microphone (M2) extérieur sont constitués pour détecter des signaux transmis au moyen de la conduction de l'air et les transmettre à l'unité (C) de commande.

9. Unité (100) audio suivant la revendication 8,
dans laquelle le microphone (M2) extérieur comprend un réseau de microphone.

10. Unité (100) audio suivant l'une des revendications précédentes,
dans laquelle l'unité (100) audio comprend une première unité de transmission sans fil, qui est constituée pour détecter des signaux électromagnétiques et les transmettre à l'unité (C) de commande et/ou pour envoyer, sous la forme de signaux électromagnétiques, des signaux reçus par l'unité (C) de commande.

11. Système audio, comprenant une première unité (100_{L} ) audio suivant l'une des revendications 1 à 10, et une deuxième unité (100_{R} ) audio suivant l'une des revendications 1 à 10.

12. Système audio suivant la revendication 11, dans lequel la première unité (100_{L} ) audio et la deuxième unité (100_{R}) audio sont constituées pour communiquer entre elles.

13. Installation (10) de résonnance magnétique ayant un système audio suivant la revendication 11 ou 12 et/ou une unité (100) audio suivant l'une des revendications 1 à 10.

14. Installation de résonnance magnétique suivant la revendication 13,
dans laquelle l'installation (10) de résonnance magnétique, le système audio et/ou l'unité (100) audio communiquent entre eux.

15. Procédé pour faire fonctionner une unité (100) audio,
dans lequel, dans une phase de fonctionnement une unité (18) à bobine de gradient d'une installation (10) de résonnance magnétique, on détecte, par une unité (IS) de capteur de vibrations de l'unité (100) audio, un signal de conduction osseuse transmis au moyen de la conduction osseuse, **caractérisé**
**en ce qu'**à l'aide du signal de conduction osseuse, on produit, par au moins un haut-parleur (S) de l'unité (100) audio, un signal de haut-parleur interférent en destruction.

16. Procédé suivant la revendication 15,
dans lequel le signal de conduction osseuse comprend plusieurs signaux de conduction osseuse, qui dépendent de la direction,
dans lequel on sépare le signal de conduction osseuse en fonction des plusieurs signaux de conduction osseuse, qui dépendent de la direction, en au moins une partie de parole et une partie de bruit.

17. Procédé suivant la revendication 16,
dans lequel, dans une phase de repos de l'unité (18) à bobine de gradient de l'installation (10) de résonnance magnétique, on produit, par la voix d'un patient (15), des signaux de conduction osseuse, qui sont détectés par l'unité (IS) de capteur de vibrations et à l'aide desquels, dans une phase de fonctionnement de l'unité (18) à bobine de gradient de l'installation (10) de résonnance magnétique, le signal de conduction osseuse est séparé en la au moins une partie de parole et une partie de bruit.

18. Procédé suivant l'une des revendications 15 à 17,
dans lequel, dans une phase de fonctionnement de l'unité (18) à bobine de gradient, on détecte, par un microphone (M1) de l'unité (100) audio, un signal acoustique intérieur à l'intérieur d'un conduit (AC) auditif,
la production du signal de haut-parleur s'effectuant à l'aide du signal acoustique intérieur.

19. Procédé suivant l'une des revendications 15 à 18,
dans lequel, dans une phase de fonctionnement de l'unité (18) à bobine de gradient, on détecte, par un microphone (M2) de l'unité (100) audio, un signal acoustique extérieur à l'extérieur d'un conduit (AC) auditif,
la production du signal de haut-parleur s'effectuant à l'aide du signal acoustique extérieur.

20. Procédé suivant la revendication 19,
dans lequel le signal acoustique extérieur comprend une parole d'instruction du patient (15).

21. Procédé suivant l'une des revendications 15 à 20,
dans lequel, dans une phase de repos de l'unité (18) à bobine de gradient de l'installation (10) de résonnance magnétique
- on détecte un signal acoustique par au moins un microphone de l'unité (100) audio, on le transmet, par une unité de transmission sans fil de l'unité (100) audio, à une unité de transmission sans fil de l'installation (10) de résonnance magnétique et on le sort par une unité de sortie et/ou
- on transmet un signal, par l'unité de transmission sans fil de l'installation (10) de résonnance magnétique, à l'unité de transmission sans fil de l'unité (100) audio et on le sort par un haut-parleur (S) de l'unité (100) audio.

22. Procédé suivant l'une des revendications 18 à 21,
dans lequel on règle une caractéristique directionnelle d'au moins un microphone (M2) à l'aide d'une activité de l'unité (18) à bobine de gradient.

23. Procédé suivant l'une des revendications 15 à 22,
dans lequel on synchronise le fonctionnement de l'unité (100) audio par une information de séquence RM.

24. Procédé suivant la revendication 23,
dans lequel, dans un laps de temps avant une vibration mécanique pronostiquée, qui est provoquée par une unité (18) à bobine de gradient d'une installation (10) de résonnance magnétique, on sort un signal de prévention.

25. Procédé suivant l'une des revendications 15 à 24,
dans lequel, par l'unité (IS) de capteur de vibration de l'unité (100) audio, on détecte un signal, au moyen duquel on détermine un déplacement et/ou une position de la tête d'un patient.
